Europäisches Patentamt
European Patent Office
Office européen des brevets

(19)

(11) Veröffentlichungsnummer: **0 273 860 A1**

(12) **EUROPÄISCHE PATENTANMELDUNG**

(21) Anmeldenummer: **87810689.7**

(22) Anmeldetag: **23.11.87**

(51) Int. Cl.⁴: **C 07 D 411/12**
C 07 D 411/14,
C 07 D 471/04,
C 07 D 491/04, A 01 N 47/36
//(C07D471/04,239:00,221:00),
(C07D491/04,307:00,239:00)

(30) Priorität: **28.11.86 CH 4770/86**

(43) Veröffentlichungstag der Anmeldung:
**06.07.88 Patentblatt 88/27**

(84) Benannte Vertragsstaaten:
**AT BE CH DE ES FR GB GR IT LI LU NL SE**

(71) Anmelder: **CIBA-GEIGY AG**
**Klybeckstrasse 141**
**CH-4002 Basel (CH)**

(72) Erfinder: **Föry, Werner, Dr.**
**Inzlingerstrasse 11**
**CH-4125 Riehen (CH)**

**Meyer, Willy**
**Talweg 49**
**CH-4125 Riehen (CH)**

Patentansprüche für folgende Vertragsstaat: ES.

(54) **N-Heterocyclosulfonyl-N'-pyrimidinyl-, N-'-triazolyl- und N'-triazinylharnstoffe.**

(57) N-(2,2-Dioxo-1,2-benzoxathiin-8-ylsulfonyl)-N'pyrimidinyl-, N'-triazolyl- oder N'-triazinyl-harnstoffe der Formel

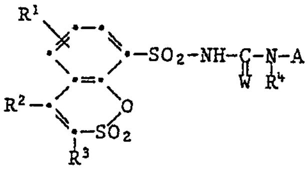

(I)

und die Salze dieser Verbindungen mit Aminen, Alkali- oder Erdalkaimetallbasen oder mit quaternären Ammoniumbasen habe gute pre-und postemergent-selektive herbizide und wuchsregulierende Eigenschaften. In dieser Formel bedeuten
$R^1$ Wasserstoff, Halogen, Nitro, $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkoxycarbonyl, $C_1$-$C_4$-Alkylthio, $C_1$-$C_4$-Alkylsulfinyl, $C_1$-$C_4$-Alkylsulfonyl oder $C_2$-$C_5$-Alkoxyalkoxy,
$R^2$ und $R^3$ unabhängig voneinander Wasserstoff oder $C_1$-$C_4$-Alkyl,
$R^4$ Wasserstoff, Methyl oder Aethyl,
W Sauerstoff oder Schwefel, und
A einen Rest

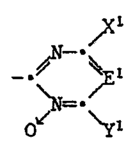
A1
,
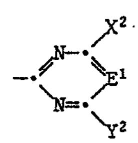
A2
,
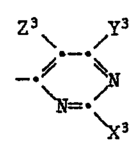
A3
,
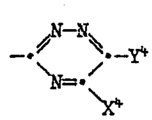
A4
,
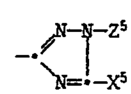
A5
,

EP 0 273 860 A1

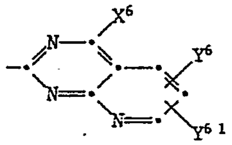

A6

,

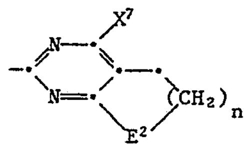

A7

oder

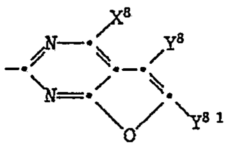

A8

bedeuten, wobei die Substituenten $E^1$, $E^2$, $X^1$, $X^2$, $X^3$, $X^4$, $X^5$, $X^6$, $X^7$, $X^8$, $Y^1$, $Y^2$, $Y^3$, $Y^4$, $Y^6$, $Y^{61}$, $Y^8$, $Y^{81}$, $Z^3$ und $Z^5$ übliche organische Reste darstellen.

**Beschreibung**

N-Heterocyclosulfonyl-N′-pyrimidinyl-, N′-triazolyl- und N′-triazinyl-harnstoffe

Die vorliegende Erfindung betrifft neue, herbizid wirksame und pflanzenwuchsregulierende N-(2,2-Dioxo-1,2-benzoxathiin-8-yl-sulfonyl)-N′-pyrimidinyl-, N′-triazolyl- oder N′-triazinyl-harnstoffe, Verfahren zu ihrer Herstellung, die sie als Wirkstoffe enthaltende Mittel sowie deren Verwendung zum Bekämpfen von Unkräutern, vor allem selektiv in Nutzpflanzenkulturen oder zum Regulieren und Hemmen des Pflanzenwachstums.

Die erfindungsgemässen Wirkstoffe entsprechen der Formel I

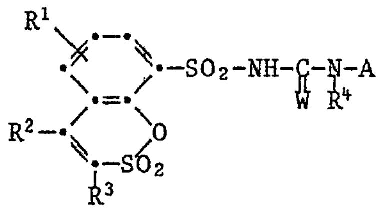

(I)

worin

$R^1$ Wasserstoff, Halogen, Nitro, $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkoxycarbonyl, $C_1$-$C_4$-Alkythio, $C_1$-$C_4$-Alkylsulfinyl, $C_1$-$C_4$-Alkylsulfonyl oder $C_2$-$C_6$-Alkoxyalkoxy,

$R^2$ und $R^3$ unabhängig voneinander Wasserstoff oder $C_1$-$C_4$-Alkyl,

$R^4$ Wasserstoff, Methyl oder Aethyl,

W Sauerstoff oder Schwefel, und

A einen Rest

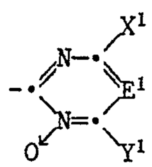

A1

,

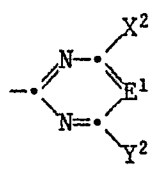

A2

,

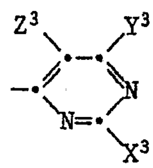

A3

,

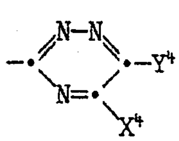

A4

,

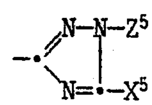

A5

,

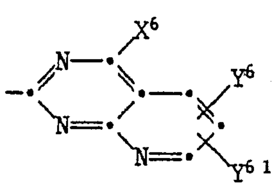

A6

,

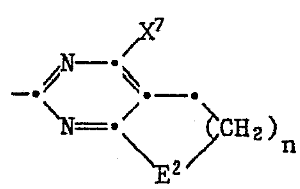

A7

oder

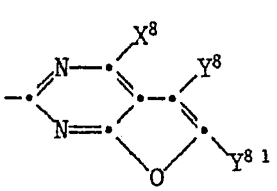

A8

bedeuten, wobei

$X^1$, $X^2$, $X^3$, $X^4$, $X^5$, $X^6$, $X^7$, $X^8$ und $Y^1$ unabhängig voneinander für Halogen, $C_1$-$C_4$-Halogenalkoxy, $C_1$-$C_4$-Halogenalkyl, $C_1$-$C_4$-Alkoxy, $C_1$-$C_4$-Alkyl, Dimethylamino, Methylamino, Aethylamino, Amino oder

$C_2$-$C_4$-Alkoxyalkyl,
$E^1$ für Stickstoff oder die Methinbrücke,
$E^2$ für Sauerstoff oder die Methylenbrücke,
$Y^2$ für Cyclopropyl, Dimethoxymethyl, Diäthoxymethyl

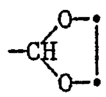 oder 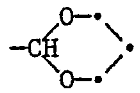 ,

$Y^3$ für Methyl, Methoxy, Aethyl, Aethoxy, $C_1$-$C_2$-Halogenalkyl, $C_1$-$C_2$-Halogenalkoxy, Dimethylamino oder Methylamino,
$Y^4$ für Wasserstoff, Methoxy, Aethoxy, Halogen oder $C_1$-$C_4$-Alkyl,
$Y^6$ und $Y^{61}$ unabhängig voneinander für Wasserstoff, $C_1$-$C_4$-Alkyl, Halogen, Methoxy oder Methylthio,
$Y^8$ und $Y^{81}$ unabhängig voneinander für Wasserstoff oder Methyl,
n für eins oder zwei,
$Z^3$ für Wasserstoff, Methyl, Aethyl, Methoxy, Aethoxy, Methoxycarbonyl, Aethoxycarbonyl, Halogen, Cyan, Nitro, Methylthio, Methylsulfinyl oder Methylsulfonyl,
$Z^3$ und $Y^3$ zusammen für eine $C_2$-$C_4$-Alkylenbrücke oder eine durch Sauerstoff unterbrochene $C_2$-$C_4$-Alkylenbrücke, und
$Z^5$ für Methyl oder Aethyl stehen,
sowie den Salzen dieser Verbindungen; mit der Massgabe, dass $R^3$ $C_1$-$C_4$-Alkyl bedeutet wenn A für einen der Reste A2 oder A5 steht.

Harnstoffverbindungen, Triazinverbindungen und Pyrimidinverbindungen mit herbizider Wirkung sind allgemein bekannt. Kürzlich wurden Sulfonylharnstoffverbindungen mit herbizider und pflanzenwuchsregulierender Wirkung, beispielsweise in den veröffentlichten europäischen Patentanmeldungen 99339 und 107979 beschrieben.

In den Definitionen ist unter Alkyl geradkettiges oder verzweigtes Alkyl zu verstehen; z.B.: Methyl, Aethyl, n-Propyl, i-Propyl oder die vier isomeren Butyle.

Unter Alkoxy ist zu verstehen: Methoxy, Aethoxy, n-Propyloxy, i-Propyloxy oder die vier isomeren Butyloxy, insbesondere aber Methoxy, Aethoxy oder i-Propyloxy.

Beispiele für Alkylthio sind Methylthio, Aethylthio, n-Propylthio, i-Propylthio oder die vier isomeren Butylthio, insbesondere aber Methylthio und Aethylthio.

Unter Halogen selbst sowie als Teil eines Substituenten wie in Halogenalkoxy, Halogenalkylthio oder Halogenalkyl sind Fluor, Chlor und Brom, vorzugsweise jedoch Fluor und Chlor zu verstehen. Halogenalkyl selbst oder als Teil von Halogenalkoxy oder Halogenalkylthio steht in der Regel für Chlormethyl, Fluormethyl, Difluormethyl, Trifluormethyl, 2-Chloräthyl, 2,2,2-Trifluoräthyl, 1,1,2,2-Tetrafluoräthyl, Pentafluoräthyl, 1,1,2-Trifluor-2-chloräthyl, 2,2,2-Trifluor-1,1-dichloräthyl, Pentachloräthyl, 3,3,3-Trifluorpropyl, 2,3-Dichlorpropyl, 1,1,2,3,3,3-Hexafluorpropyl, insbesondere aber Fluormethyl, Chlormethyl, Difluormethyl und Trifluormethyl.

Beispiele für Alkoxyalkyl sind: Methoxymethyl, Methoxyäthyl, Methoxypropyl, Aethoxyäthyl, Aethoxymethyl oder Propyloxymethyl. Beispiele für Alkoxyalkoxy sind: Methoxymethoxy, Methoxyäthoxy, Methoxypropyloxy, Aethoxymethoxy, Aethoxyäthoxy sowie Propyloxymethoxy. Alkylengruppen sind im Rahmen der Definition unter Formel I Aethylen, Propylen, Butylen, 1-Methyläthylen, 1-Aethyläthylen, 2-Methylbutylen, 1-Methylbutylen oder wenn die Brücke durch Sauerstoff unterbrochen ist auch $-CH_2-O-CH_2-$ oder $-CH_2-O-CH_2-CH_2-$.

Die Erfindung umfasst ebenfalls die Salze, die die Verbindungen der Formel I mit Aminen, Alkali- und Erdalkalimetallbasen oder quaternären Ammoniumbasen bilden können.

Unter Alkali- und Erdalkalimetallhydroxiden als Salzbildner sind die Hydroxide von Lithium, Natrium, Kalium, Magnesium oder Calcium hervorzuheben, insbesondere aber die von Natrium oder Kalium.

Beispiele für zur Salzbildung geeigneter Amine sind primäre, sekundäre und tertiäre aliphatische und aromatische Amine wie Methylamin, Aethylamin, Propylamin, i-Propylamin, die vier isomeren Butylamine, Dimethylamin, Diäthylamin, Diäthanolamin, Dipropylamin, Diisopropylamin, Di-n-butylamin, Pyrrolidin, Piperidin, Morpholin, Trimethylamin, Trähylamin, Tripropylamin, Chinuclidin, Pyridin, Chinolin und i-Chinolin, insbesondere aber Aethyl-, Propyl-, Diäthyl- oder Triäthylamin, vor allem aber iso-Propylamin, Diäthanolamin und 1,4-Diazabicyclo[2.2.2]octan.

Beispiele für quaternäre Ammoniumbasen sind im allgemeinen die Kationen von Halogenammoniumsalzen, z.B. das Tetramethylammoniumkation, das Tetraäthylammoniumkation, das Trimethyläthylammoniumkation, aber auch das Ammoniumkation.

Unter den Verbindungen der Formel I sind diejenigen bevorzugt, in denen entweder

a) W Sauerstoff ist oder
b) $R^1$ und $R^2$ für Wasserstoff, und $R^3$ für Methyl stehen oder
c) $R^4$ für Wasserstoff steht.

Als weitere hervorzuhebende Untergruppe von Verbindungen der Formel I ist diejenige zu nennen, in denen W für Sauerstoff und $R^4$ für Wasserstoff stehen.

Besonders bevorzugte Untergruppen von Verbindungen der Formel I sind dadurch charakterisiert, dass entweder

aa) W für Sauerstoff, $R^4$ für Wasserstoff und A für die Gruppe A1 stehen, wobei $X^1$ und $Y^1$ unabhängig voneinander je $C_1$-$C_4$-Alkyl oder $C_1$-$C_4$-Alkoxy bedeutet, oder

bb) W für Sauerstoff, $R^4$ für Wasserstoff und A für die Gruppe A2 stehen, wobei $Y^2$ Cyclopropyl bedeutet, oder

cc) W für Sauerstoff, $R^4$ für Wasserstoff und A für die Gruppe A3 stehen, wobei $Z^3$ Halogen, Methyl oder Aethyl bedeutet, oder

dd) W für Sauerstoff, $R^4$ für Wasserstoff und A für die Gruppe $A^3$ stehen, wobei $Z^3$ Halogen und $Y^3$ $C_1$-$C_2$-Alkoxy oder $C_1$-$C_2$-Alkyl bedeuten, oder

ee) W für Sauerstoff, $R^4$ für Wasserstoff und A für die Gruppe A5 stehen, wobei $X^5$ $C_1$-$C_4$-Alkoxy und $Z^5$ Methyl oder Aethyl bedeuten, oder

ff) W für Sauerstoff, $R^4$ für Wasserstoff und A für die Gruppe A4 stehen, wobei $X^4$ $C_1$-$C_4$-Alkyl oder $C_1$-$C_4$-Alkoxy bedeutet.

Als bevorzugte Einzelverbindungen der Formel I sind zu nennen:
N-(3-Methyl-2,2-dioxo-1,2-benzoxathiin-8-ylsulfonyl)-N'-(2,6-dimethoxy-5-chlor-pyrimidin-4-yl)-harnstoff und
N-(3-Methyl-2,2-dioxo-1,2-benzoxathiin-8-ylsulfonyl)-N'-(2-methoxy-5-brom-6-methylpyrimidin-4-yl)-harnstoff.

Die Herstellung der Verbindungen der Formel I erfolgt im allgemeinen nach den folgenden Methoden.

Nach einem ersten Verfahren werden die Verbindungen der Formel I erhalten, indem man ein Sulfonamid der Formel II

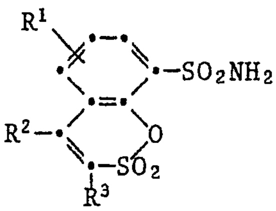

(II),

worin $R^1$, $R^2$ und $R^3$ die unter Formel I gegebene Bedeutung haben, in Gegenwart einer Base mit einem Carbamat der Formel III

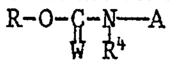

(III),

worin $R^4$, A und W die unter Formel I gegebene Bedeutung haben und R für Phenyl, $C_1$-$C_4$-Alkyl oder substituiertes Phenyl steht, umsetzt.

Nach einem zweiten Verfahren gelangt man zu Verbindungen der Formel I, indem man ein Sulfonylcarbamat der Formel IV

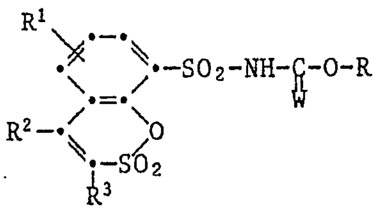

(IV),

worin $R^1$, $R^2$, $R^3$ und W die unter Formel I gegebene Bedeutung haben und R für Phenyl, $C_1$-$C_4$-Alkyl oder substituiertes Phenyl steht, mit einem Amin der Formel V

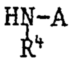

(V),

worin A und $R^4$ die unter Formel I gegebene Bedeutung haben, umsetzt.

Schliesslich kann man die Verbindungen der Formel I auch erhalten, indem man ein Sulfonylisocyanat der Formel VI

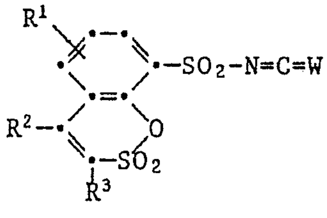

(VI)

worin $R^1$, $R^2$, $R^3$ und W die unter Formel I gegebenen Bedeutungen haben, mit einem Amin der oben angegebenen Formel V umsetzt.

Die erhaltenen Harnstoffe der Formel I können gewünschtenfalls mittels Aminen, Alkalimetall- oder Erdalkalimetallhydroxiden oder quaternären Ammoniumbasen in Additionssalze überführt werden. Dieses geschieht beispielsweise durch Umsetzen mit der äquimolaren Menge Base und Verdampfen der Lösungsmittels.

Die Umsetzungen zu Verbindungen der Formel I werden vorteilhafterweise in aprotischen, inerten organischen Lösungsmitteln vorgenommen. Solche Lösungsmittel sind Kohlenwasserstoffe wie Benzol, Toluol, Xylol oder Cyclohexan, Tetrachlorkohlenstoff, oder Chorbenzol, Aether wie Diäthyläther, Aethylenglykoldimethyläther, Diäthylenglykoldimethyläther, Tetrahydrofuran oder Dioxan, Nitrile wie Acetonitril oder Propionitril, Amide wie Dimethylformamid, Diäthylformamid oder N-Methylpyrrolidinon. Die Reaktionstemperaturen liegen vorzugsweise zwischen -20° und +120° C. Die Umsetzungen verlaufen im allgemeinen leicht exotherm und können bei Raumtemperatur durchgeführt werden. Zwecks Abkürzung der Reaktionszeit oder auch zum Einleiten der Umsetzung wird zweckdienlich für kurze Zeit bis zum Siedepunkt des Reaktionsgemisches aufgewärmt. Die Reaktionszeiten können ebenfalls durch Zugabe einiger Tropfen Base als Reaktionskatalysator verkürzt werden. Als Basen sind insbesondere tertiäre Amine wie Trimethylamin, Triäthylamin, Chinuclidin, 1,4-Diazabicyclo[2.2.2]octan, 1,5-Diazabicyclo[4.3.0]non-5-en oder 1,8-Diazabicyclo[5.4.0]undec-7-en geeignet. Als Basen können aber auch anorganische Basen wie Hydride wie Natrium-oder Calcicumhydrid, Hydroxide wie Natrium- und Kaliumhydroxid, Carbonate wie Natrium- und Kaliumcarbonat oder Hydrogencarbonate wie Kalium-und Natriumhydrogencarbonat verwendet werden.

Die Endprodukte der Formel I können durch Einengen und/oder Verdampfen des Lösungsmittels isoliert und durch Umkristallisieren oder Zerreiben des festen Rückstandes in Lösungsmitteln, in denen sie sich nicht gut lösen, wie Aether, aromatischen Kohlenwasserstoffen oder chlorierten Kohlenwasserstoffen gereinigt werden.

Die Zwischenprodukte der Formeln II, IV und VI und ihre Herstellung sind aus der EP-A-128116 bzw. EP-A-177448 bekannt.

Die als Ausgangsmaterialien verwendeten Aminopyrimidine, Aminotriazole und Aminotriazine der Formel V sowie entsprechende Carbamate der Formel III sind entweder bekannt oder sie lassen sich nach bekannten und in der Literatur beschriebenen Methoden erhalten.

Die Wirkstoffe der Formel I sind stabile Verbindungen. Ihre Handhabung bedarf keiner vorsorglichen Massnahmen.

Bei geringeren Aufwandmengen zeichnen sich die Verbindungen der Formel I durch gute selektiv-wuchshemmende und selektiv-herbizide Eigenschaften aus, die sie ausgezeichnet zum Einsatz in Kulturen von Nutzpflanzen, insbesondere in Getreide, Reis, Baumwolle, Soja, Zuckerrüben und Mais befähigen. Es werden dabei teilweise auch Unkräuter geschädigt, welchen bisher nur mit Totalherbiziden beizukommen war.

Die Wirkungsart dieser Wirkstoffe ist unüblich. Viele sind translozierbar, d.h. sie werden von der Pflanze aufgenommen und an andere Stellen transportiert, wo sie dann zur Wirkung kommen. So gelingt es beispielsweise, durch Oberflächenbehandlung perennierende Unkräuter bis in die Wurzeln zu schädigen. Die neuen Verbindungen der Formel I wirken bereits bei - im Vergleich zu anderen Herbiziden und Wuchsregulatoren - sehr geringen Aufwandmengen.

Die Verbindungen der Formel I haben ausserdem starke pflanzenwuchsregulierende, insbesondere pflanzenwuchshemmende, Eigenschaften. Es werden sowohl Monokotyledonen als auch Dikotyledonen in ihrem Wachstum beeinträchtigt.

So können z.B. die in der Landwirtschaft in tropischen Gegenden häufig als "cover crops" (Bodenbedecker) angepflanzte Leguminosen durch die Verbindungen der Formel I in ihrem Wachstum selektiv gehemmt werden, so dass zwar die Bodenerosion zwischen den Kulturpflanzen verhindert wird, die "cover crops" jedoch nicht zur Konkurrenz für die Kultur werden können.

Eine Hemmung des vegetativen Wachstums erlaubt bei vielen Kulturpflanzen eine dichtere Anpflanzung der Kultur, so dass ein Mehrertrag, bezogen auf die Bodenfläche, erzielt werden kann.

Ein weiterer Mechanismus der Ertragssteigerung mit Wachstumshemmern beruht darauf, dass die Nährstoffe in stärkerem Masse der Blüten-und Fruchtbildung zugute kommen, während das vegetative Wachstum eingeschränkt wird.

Bei grösseren Aufwandmengen an Wirkstoffen der Formel I werden alle getesteten Pflanzen in ihrer Entwicklung so geschädigt, dass sie absterben.

Die Erfindung betrifft auch herbizide und pflanzenwachstumsregulierende Mittel, welche einen neuen Wirkstoff der Formel I enthalten, sowie Verfahren zur pre- und post-emergenten Unkrautbekämpfung und zur Hemmung des Pflanzenwuchses von monokotylen und dikotylen Pflanzen, insbesondere Gräsern, tropischen Bodenbedeckern und Tabakgeiztrieben.

Die Verbindungen der Formel I werden in unveränderter Form oder vorzugsweise als Mittel zusammen mit den in der Formulierungstechnik üblichen Hilfsmitteln eingesetzt und werden daher z.B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen in bekannter Weise verarbeitet. Die Anwendungsverfahren wie Versprühen, Vernebeln, Verstäuben, Verstreuen oder Giessen werden gleich wie die Art der Mittel den angestrebten Zielen und den gegebenen Verhältnissen entsprechend gewählt.

In vorteilhafter Weise können die erfindungsgemässen Wirkstoffe oder Mittel auch auf das Vermehrungsgut der Kulturpflanze aufgebracht werden. Besonders zu erwähnen ist hier die Samenbeizung. Vermehrungsgut

sind Samen, Stecklinge oder sonstige Teile der Pflanze, aus denen die Kulturpflanze gezogen werden kann. Das mit einer pflanzenwuchsregulatorisch oder herbizid wirksamen Menge einer Verbindung der Formel I behandelte Vermehrungsgut ist ebenfalls Gegenstand der Erfindung.

Die Formulierungen, d.h. die den Wirkstoff der Formel I und gegebenenfalls einen festen oder flüssigen Zusatzstoff enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Aether und Ester, wie Aethanol, Aethylenglykol, Aethylenglykolmonomethyl- oder -äthyläther, Ketone wie Cyclohexanon, stark polare Lösungsmittel wie N-Methyl-2-pyrrolidon, Dimethylsulfoxid oder Dimethylformamid, sowie gegebenenfalls epoxidierte Pflanzenöle, wie epoxidiertes Kokosnussöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z.B. Bimsstein, Ziegelbruch, Sepiolit oder Bentonit, als nicht sorptive Trägermaterialien z.B. Calcit oder Sand in Frage. Darüberhinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach der Art des zu formulierenden Wirkstoffes der Formel I nichtionogene, kation-und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen als auch wasserlösliche synthetische oberflächenaktive Verbindungen sein.

Als Seifen seien die Alkali-, Erdalkali- oder gegebenenfalls substituierte Ammoniumsalze von höheren Fettsäuren ($C_{10}$-$C_{22}$), wie z.B. die Na- oder K-Salze der Oel- oder Stearinsäure, oder von natürlichen Fettsäuregemischen, die z.B. aus Kokosnuss- oder Talgöl gewonnen werden können, genannt. Ferner sind auch die Fettsäuremethyl-taurinsalze zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxid-Addukten. Die sulfonierten Benzimidazolderivate enthalten vorzugsweise 2-Sulfonsäuregruppen und einen Fettsäurerest mit 8 bis 22 C-Atomen. Alkylarylsulfonate sind z.B. die Na-, Ca- oder Triäthanolaminsalze der Dodecylbenzolsulfonsäure, der Dibutylnaphthalinsulfonsäure oder eines Naphthalinsulfonsäure-Formaldehydkondensationsproduktes.

Ferner kommen auch entsprechende Phosphate wie z.B. Salze des Phosphorsäureesters eines p-Nonylphenol-(4-14)-Aethylenoxid-Adduktes oder Phospholipide in Frage.

Als nicht ionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 10 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Aethylenglykoläthergruppen und 10 bis 100 Propylenglykoläthergruppen enthaltenden Polyäthylenoxidaddukte an Polypropylenglykol, Aethylendiaminopolypropylenglykol und Alkylpolypropylenglykol mit 1 bis 10 Kohlenstoffatomen in der Alkylkette, die genannten Verbindungen enthalten üblicherweise pro Propylenglykol-Einheit 1 bis 5 Aethylenglykoleinheiten.

Als Beispiele nichtionischer Tenside seien Nonylphenolpolyäthoxyäthanole, Ricinusölpolyglykoläther, Polypropylen-Polyäthylenoxid-addukte, Tributylphenoxypolyäthoxyäthanol, Polyäthylenglykol und Octylphenoxypolyäthoxyäthanol erwähnt.

Ferner kommen auch Fettsäureester von Polyoxyäthylensorbitan wie das Polyoxyäthylensorbitan-trioleat in Betracht.

Bei den kationischen Tensiden handelt es sich vor allem um quaternäre Ammoniumsalze, welche als N-Substituenten mindestens einen Alkylrest mit 8 bis 22 C-Atomen enthalten und als weitere Substituenten niederige, gegebenenfalls halogenierte Alkyl-, Benzyl-oder niedrige Hydroxyalkylreste aufweisen. Die Salze liegen vorzugsweise als Halogenide, Methylsulfate oder Aethylsulfate vor, z.B. das Stearyltrimethylammoniumchlorid oder das Benzyldi(2-chloräthyl)-äthylammoniumbromid.

Die in der Formulierungstechnik gebräuchlichen Tenside sind u.a. in folgenden Publikationen beschrieben:
"1986 International Mc Cutcheon's Emulsifiers & Detergents" Glen Rock, New Jersey USA;
H. Stache, "Tenside-Taschenbuch", 2. Aufl., C. Hanser Verlag, München, Wien, 1981;
M. and J. Ash. "Encyclopedia of Surfactants", Vol. I-III, Chemical Publishing Co., New York, 1980-1981.

Die pestiziden Zubereitungen enthalten in der Regel 0,1 bis 95 %, insbesondere 0,1 bis 80 % Wirkstoff der Formel I, 1 bis 99,9 % eines festen oder flüssigen Zusatzstoffes und 0 bis 25 %, insbesondere 0,1 bis 25 % eines Tensides.

Insbesondere setzen sich bevorzugte Formulierungen folgendermassen zusammen: (% = Gewichtsprozent).

Emulgierbare Konzentrate:
Aktiver Wirkstoff: 1 bis 20 %, bevorzugt 5 bis 10 %
oberflächenaktive Mittel: 5 bis 30 %, vorzugsweise 10 bis 20 %
flüssiges Trägermittel: 50 bis 94 %, vorzugsweise 70 bis 85 %

Stäube:
Aktiver Wirkstoff: 0,1 bis 10 %, vorzugsweise 0,1 bis 1 %
festes Trägermittel: 99,9 bis 90 %, vorzugsweise 99,9 bis 99 %

Suspensions-Konzentrate:
Aktiver Wirkstoff: 5 bis 75 %, vorzugsweise 10 bis 50 %
Wasser: 94 bis 24 %, vorzugsweise 88 bis 30 %
oberflächenaktives Mittel: 1 bis 40 %, vorzugsweise 2 bis 30%

Benetzbares Pulver:
Aktiver Wirkstoff: 0,5 bis 90 %, vorzugsweise 1 bis 80 %
oberflächenaktives Mittel: 0,5 bis 20 %, vorzugsweise 1 bis 15 %
festes Trägermittel: 5 bis 95 %, vorzugsweise 15 bis 90 %

Granulate:
Aktiver Wirkstoff: 0,5 bis 30 %, vorzugsweise 3 bis 15 %
festes Trägermittel: 99,5 bis 70 %, vorzugsweise 97 bis 85 %.

Während als Handelsware ehere konzentrierte Mittel bevorzugt werden, verwendet der Endverbraucher in der Regel verdünnte Mittel. Die Anwendungsformen können bis hinab zu 0,001 % an Wirkstoff verdünnt werden. Die Aufwandmengen betragen in der Regel 0,01 bis 10 kg AS/ha, vorzugsweise 0,025 bis 5 kg AS/ha.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Herstellungsbeispiel H1

N-(3-Methyl-2,2-dioxo-1,2-benzoxathiin-8-yl-sulfonyl)-N'-)2,6-dimethoxy-5-chlor-pyrimidin-4-yl)harnstoff (Verbindung 3.01)

a) N-(2,6-Dimethoxy-5-chlor-pyrimidin-2-yl)-phenyl-carbamat.

Eine Lösung von 7 g Diphenylcarbonat in 45 ml trockenem Dimethylformamid wird bei 10°C innerhalb 6 Minuten zu 1,3 g einer Natriumhydrid-Oel-Suspension (55 %) in 15 ml trockenem Dimethylformamid getropft. Zu dieser Mischung wird bei 15°C während 30 Minuten eine Lösung von 5,68 g 4-Amino-2,6-dimethoxy-5-chlorpyrimidin in 12 ml Dimethylformamid getropft und während 40 Minuten bei dieser Temperatur ausgerührt. Nach zweimaliger Zugabe von 0,3 ml Essigsäure wird die Reaktionsmischung über Hyflo filtriert, in eine Mischung von 180 ml Essigester, 180 ml Eis-Wasser, 20 ml 2N Salzsäure eingerührt, mit Eis-Wasser und Essigester gewaschen, die organische Phase über Natriumsulfat getrocknet und am Vakuum eingedampft. 3,3 g des gewünschten Carbamates kristallisieren aus dem öligen Rückstand nach Aetherzusatz bei 0° über Nacht mit Smp: 98-99°C, und werden ohne weitere Reinigungsschritte weiterverarbeitet.

b) 3,1 g des rohen N-(2,6-Dimethoxy-5-chlorpyrimidin-2-yl)-phenylcarbamats werden zu einer Lösung von 2,09 g 3-Methyl-2,2-dioxo-1,2-benzoxathiin-8-yl-sulfonamid, 1,195 ml Diazabicyclo[5.4.0]undec-5-en in 33 ml trockenem Dioxan gegeben. Nach einstündigem Ausrühren bei Raumtemperatur wird mit 5 ml 2N Salzsäure + 60 ml Wasser ausgefällt und filtriert. Man erhält 2,2 g N-(3-Methyl-2,2-dioxo-1,2-benzoxathiin-8-yl-sulfonyl)-N'-(2,6-dimethoxy-5-chlor-pyrimidin-4-yl)-harnstoff mit dem Schmelzpunkt 236-237°C.

Nach diesem oder den in der Beschreibung angegebenen Verfahren sind nachfolgende Verbindungen der Tabelle 1 bis 8 herstellbar:

Tabelle 1:

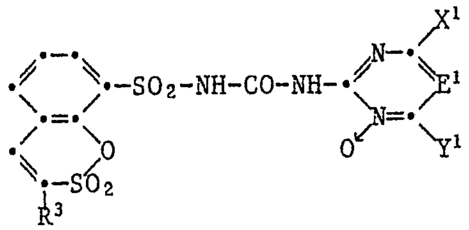

| Verb. Nr. | $E^1$ | $X^1$ | $Y^1$ | $R^3$ | Smp. [°C] |
|---|---|---|---|---|---|
| 1.01 | CH | $CH_3$ | $OCH_3$ | $CH_3$ | |
| 1.02 | CH | $CH_3$ | $CH_3$ | $CH_3$ | 189-90 |
| 1.03 | N | $CH_3$ | $CH_3$ | $CH_3$ | |
| 1.04 | CH | $CH_3$ | $OC_2H_5$ | $CH_3$ | |
| 1.05 | CH | $OCH_3$ | $OCH_3$ | $CH_3$ | |
| 1.06 | CH | $N(CH_3)_2$ | $OCH_3$ | $CH_3$ | |
| 1.07 | CH | $CH_3$ | $CH_3$ | H | |

Tabelle 2:

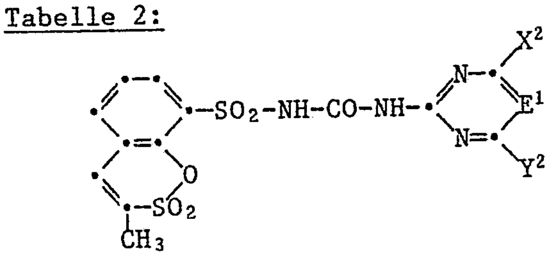

| Verb. Nr. | $E^1$ | $X^2$ | $Y^2$ | Smp. [°C] |
|---|---|---|---|---|
| 2.01 | CH | $CH_3$ | Cyclopropyl | 232-34 |
| 2.02 | N | $OCH_3$ | Cyclopropyl | |
| 2.03 | CH | $OCH_3$ | Cyclopropyl | |
| 2.04 | N | $CH_3$ | Cyclopropyl | |

Tabelle 3:

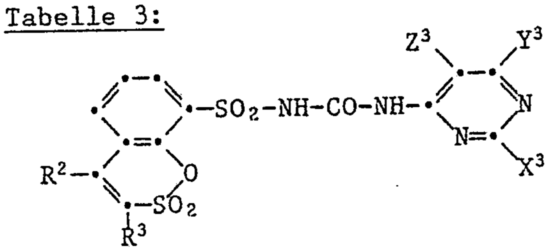

| Verb. Nr. | $X^3$ | $Y^3$ | $Z^3$ | $R^3$ | Smp. [°C] | $R^2$ |
|---|---|---|---|---|---|---|
| 3.01 | $OCH_3$ | $OCH_3$ | Cl | $CH_3$ | 236-237 | H |
| 3.02 | $CH_3$ | $OCH_3$ | Cl | $CH_3$ | 232-4 | H |
| 3.03 | $OCH_3$ | $CH_3$ | Br | $CH_3$ | 210-11 | H |
| 3.04 | $OCH_3$ | $-CH_2-CH_2-CH_2-$ | | $CH_3$ | 218-9 | H |
| 3.05 | $OCH_3$ | $OCH_3$ | $CH_3$ | $CH_3$ | | H |
| 3.06 | $OCH_3$ | $OCH_3$ | F | $CH_3$ | 241-42 | H |
| 3.07 | $OCH_3$ | $N(CH_3)_2$ | Cl | $CH_3$ | | H |
| 3.08 | $OCH_3$ | $OCH_3$ | $SCH_3$ | $CH_3$ | 238-39 | H |
| 3.09 | $OCH_3$ | $OCH_3$ | $SOCH_3$ | $CH_3$ | | H |
| 3.10 | $OCH_3$ | $OCH_3$ | $SO_2CH_3$ | $CH_3$ | | H |
| 3.11 | $OCH_3$ | $CH_3$ | Br | H | 175-77 | H |
| 3.12 | $OCH_3$ | $OCH_3$ | $CH_3$ | H | 214-15 | H |
| 3.13 | $OCH_3$ | $OCH_3$ | Cl | $C_2H_5$ | | H |
| 3.14 | $OCH_3$ | $OCH_3$ | Cl | $CH_3$ | | $CH_3$ |
| 3.15 | $OCH_3$ | $OCH_3$ | Cl | H | | $CH_3$ |

Tabelle 4:

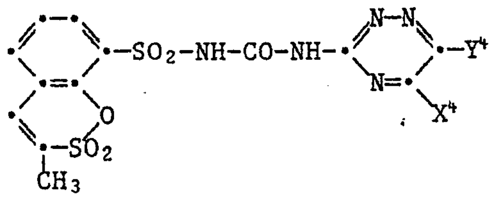

| Verb. Nr. | $X^4$ | $Y^4$ | Smp. [°C] |
|---|---|---|---|
| 4.01 | $CH_3$ | $CH_3$ | |
| 4.02 | $CH_3$ | H | |
| 4.03 | $OCH_3$ | H | |
| 4.04 | $OCH_3$ | $OCH_3$ | |

Tabelle 5:

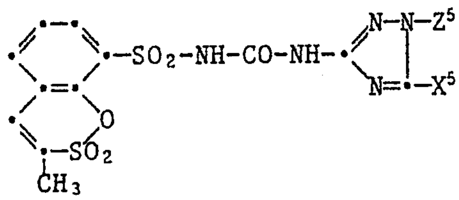

| Verb. Nr. | $X^5$ | $Z^5$ | Smp. [°C] |
|---|---|---|---|
| 5.01 | $OCH_3$ | $CH_3$ | |
| 5.02 | $CH_3$ | $CH_3$ | |

Tabelle 6:

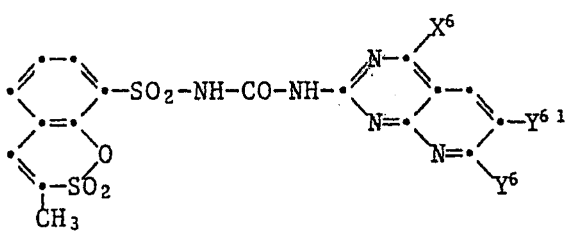

| Verb. Nr. | $X^6$ | $Y^6$ | $Y^{6\,1}$ | Smp. [°C] |
|---|---|---|---|---|
| 6.01 | $OCH_3$ | $CH_3$ | H | |
| 6.02 | $OCH_3$ | H | $CH_3$ | |
| 6.03 | $CH_3$ | $CH_3$ | H | |

Tabelle 7:

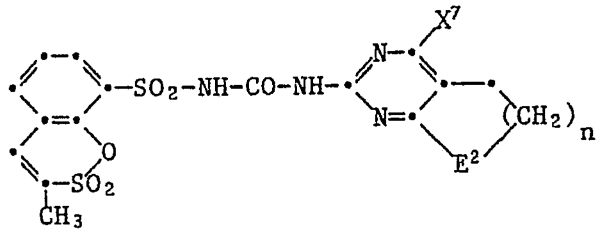

| Verb. Nr. | $E^2$ | n | $X^7$ | Smp. [°C] |
|---|---|---|---|---|
| 7.01 | O | 1 | $CH_3$ | |
| 7.02 | O | 1 | $OCH_3$ | |
| 7.03 | $CH_2$ | 1 | $OCH_3$ | |
| 7.04 | O | 2 | $OCH_3$ | |
| 7.05 | $CH_2$ | 2 | $OCH_3$ | |
| 7.06 | $CH_2$ | 1 | $CH_3$ | |

Tabelle 8:

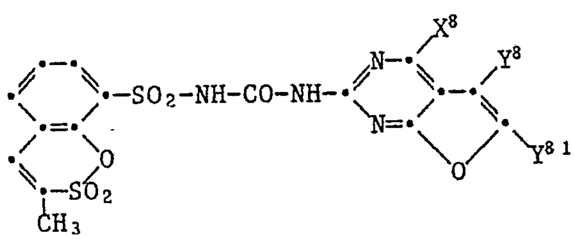

| Verb. Nr. | $X^8$ | $Y^8$ | $Y^{81}$ | Smp. [°C] |
|---|---|---|---|---|
| 8.01 | $CH_3$ | H | $CH_3$ | |
| 8.02 | $OCH_3$ | H | $CH_3$ | |
| 8.03 | $OCH_3$ | H | H | |
| 8.04 | $CH_3$ | H | $CH_3$ | |
| 8.05 | $OCH_3$ | $CH_3$ | $CH_3$ | |

Formulierungsbeispiele:

Beispiel F 1: Formulierungsbeispiele für Wirkstoffe der Formel I (% = Gewichtsprozent)

| a) Spritzpulver | a) | b) | c) |
|---|---|---|---|
| Wirkstoff gemäss Tabelle 1 bis 8 | 20 % | 50 % | 0,5 % |
| Na-Ligninsulfonat | 5 % | 5 % | 5 % |
| Na-Laurylsulfat | 3 % | - | - |
| Na-Diisobutylnaphthalinsulfonat | - | 6 % | 6 % |
| Octylphenolpolyäthylenglykol-äther /7-8 Mol AeO) | - | 2 % | 2 % |
| Hochdisperse Kieselsäure | 5 % | 17 % | 27 % |
| Kaolin | 67 % | 20 % | - |
| Natriumchlorid | - | - | 59,5 % |

Der Wirkstoff wird mit den Zusatstoffen gut vermischt und in einer geeigneten Mühle gut vermahlen. Man erhält Spritzpulver, die sich mit Wasser zu Suspensionen jeder gewünschten Konzentration verdünnen lassen.

| b) Emulsions-Konzentrat | a) | b) |
|---|---|---|
| Wirkstoff gemäss Tabelle 1 bis 8 | 10 % | 1 % |
| Octylphenolpolyäthylenglykol-äther (4-5 Mol AeO) | 3 % | 3 % |
| Ca-Dodecylbenzolsulfonat | 3 % | 3 % |
| Ricinusölpolyglykoläther (36 Mol AeO) | 4 % | 4 % |
| Cyclohexanon | 30 % | 10 % |
| Xylolgemisch | 50 % | 79 % |

Aus diesem Konzentrat können durch Verdünnen mit Wasser Emulsionen jeder gewünschten Konzentration hergestellt werden.

| c) Stäubemittel | a) | b) |
|---|---|---|
| Wirkstoff gemäss Tabelle 1 bis 8 | 0,1 % | 1 % |
| Talkum | 99,9 % | - |
| Kaolin | - | 99 % |

Man erhält anwendungsfertige Stäubemittel, indem der Wirkstoff mit dem Träger vermischt und auf einer geeigneten Mühle vermahlen wird.

d) Extruder Granulat

| | a) | b) |
|---|---|---|
| Wirkstoff gemäss Tabelle 1 bis 8 | 10 % | 1 % |
| Na-Ligninsulfonat | 2 % | 2 % |
| Carboxymethylcellulose | 1 % | 1 % |
| Kaolin | 87 % | 96 % |

Der Wirkstoff wird mit den Zusatzstoffen vermischt, vermahlen und mit Wasser angefeuchtet. Dieses Gemisch wird extrudiert und anschliessend im Luftstrom getrocknet.

e) Umhüllungs-Granulat
Wirkstoff gemäss Tabelle 1 bis 8 3 %
Polyäthylenglykol (MG 200) 3 %
Kaolin 94 %

Der Wirkstoff wird in einem Mischer auf das mit Polyäthylenglykol angefeuchtete Kaolin gleichmässig aufgetragen. Auf diese Weise erhält man staubfreie Umhüllungs-Granulate.

f) Suspensions-Konzentrat

| | a) | b) |
|---|---|---|
| Wirkstoff gemäss Tabelle 1 bis 8 | 40 % | 5 % |
| Aethylenglykol | 10 % | 10 % |
| Nonylphenolpolyäthylenglykoläther (15 Mol AeO) | 6 % | 1 % |
| Na-Ligninsulfonat | 10 % | 5 % |
| Carboxymethylcellulose | 1 % | 1 % |
| 37 %ige wässrige Formaldehyd-Lösung | 0,2 % | 0,2 % |
| Silikonöl in Form einer 75 %igen wässrigen Emulsion | 0,8 % | 0,8 % |
| Wasser | 32 % | 77 % |

Der Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

g) Salzlösung Wirkstoff gemäss Tabelle 1 bis 8 5 %
Isopropylamin 1 %
Octylphenolpolyäthylenglykoläther
(78 Mol AeO) 3 %
Wasser 91 %

Biologische Beispiele:

Beispiel B 1: Herbizidwirkung vor dem Auflaufen der Pflanzen

Kunststofftöpfe werden mit expandiertem Vermiculit (Dichte: 0,135 g/cm$^3$, Wassesrabsorptionsvermögen: 0,565 1/1) gefüllt. Nach dem Sättigen des nicht adsorptiven Vermiculits mit einer wässrigen Wirkstoffemulsion in deionisiertem Wasser, die die Wirkstoffe in einer Konzentration von 70,8 ppm enthält, werden Samen der folgenden Pflanzen auf die Oberfläche gesät: Nasturtium officinalis, Agrostis tenuis und Stellaria media. Die Versuchsgefässe werden anschliessend in einer Klimakammer bei 20°C, einer Beleuchtung von ca. 30 kLux und einer relativen Luftfeuchtigkeit von 70 % gehalten. Während einer Keimphase von 4 bis 5 Tagen werden die Töpfe zur Erhöhung der örtlichen Luftfeuchtigkeit mit lichtdurchlässigem Material abgedeckt und mit deionisiertem Wasser gegossen. Nach dem 5. Tag wird dem Giesswasser 0,5 % eines handelsüblichen Flüssigdüngers (®Greenzit, ex Ciba-Geigy) zugesetzt. 12 Tage nach der Aussaat wird der Versuch ausgewertet.

In diesem Versuch zeigen die Verbindungen der Formel I gemäss Tabelle 1 bis 8 gute Herbizidwirkung.

Beispiel B 2: Wuchshemmung bei tropischen Bodenbedecker-Leguminosen (cover crops).

Die Versuchspflanzen (centrosema plumieri und centrosema pubescens) werden bis zum ausgewachsenen Stadium herangezogen und bis auf eine Höhe von 60 cm zurückgeschnitten. Nach 7 Tagen wird der Wirkstoff als wässrige Emulsion gespritzt. Die Versuchspflanzen werden bei 70 % relativer Luftfeuchtigkeit und 600 lux Kunstlicht, pro Tag 14 Stunden, bei Temperaturen von 27° bei Tag und 21°C bei Nacht gehalten. 4 Wochen nach der Applikation wird der Versuch ausgewertet. Es werden dabei der Neuzuwachs im Vergleich zur Kontrolle abgeschätzt und gewogen und die Phytotoxizität bewertet. In diesem Versuch zeigen die mit den Wirkstoffen der Formel I gemäss Tabelle 1 bis 8 behandelten Pflanzen eine deutliche Reduktion des Neuzuwachses (weniger als 20 % des Neuzuwachses bei unbehandelten Kontrollpflanzen), ohne dass dabei die Versuchspflanzen geschädigt werden.

Beispiel B 3: Wuchsregulierung an Sojabohnen

In Kunststoffbehältern mit einem Erde-Torf-Sandgemisch im Verhältnis 6:3:1 werden Sojabohnen der Sorte "Hark" angesät und in eine Klimakammer gegeben. Durch optimale Temperaturwahl, Beleuchtung, Dünger zugabe und Bewässerung entwickeln sich die Pflanzen nach ca. 5 Wochen bis zum 5-6 Trifolia-Blattstadium. Zu diesem Zeitpunkt werden die Pflanzen mit der wässrigen Brühe eines Wirkstoffs der Formel I gemäss Tabelle 1 bis 8 bis zur guten Benetzung besprüht. Die Wirkstoffkonzentration beträgt bis zu 100 g AS/ha. Die Auswertung erfolgt ca. 5 Wochen nach der Applikation des Wirkstoffs. Im Vergleich zu unbehandelten Kontrollpflanzen bewirken die erfindungsgemässen Wirkstoffe der Formel I eine merkliche Erhöhung der Anzahl und des Gewichts der Schoten im Haupttrieb.

Beispiel B 4: Wuchshemmung bei Getreide

In Kunststofftöpfen mit sterilisierter Erde werden in Getreidearten Hordeum vulgare (Sommergerste) und Secale (Sommerroggen) im Gewächshaus angesät und nach Bedarf bewässert. Die Sprösslinge werden ca. 21 Tage nach der Aussaat mit der wässrigen Spritzbrühe eines Wirkstoffes der Formel I gemäss Tabelle 1 bis 8 besprüht. Die Wirkstoffmenge beträgt bis zu 100 g Aktivsubstanz pro Hektar. 21 Tage nach Applikation wird das Wachstum des Getreides beurteilt. Die behandelten Pflanzen weisen im Vergleich zu unbehandelten Kontrollen eine Verringerung des Neuzuwachses (60-90 % der Kontrolle), sowie teilweise eine Zunahme der Stengeldurchmesser auf.

Beispiel B 5: Wuchshemmung bei Gräsern

In Kunststoffschalen mit Erde-Toft-Sand-Gemisch (6:3:1) werden die Gräser Lolium perenne, Poa pratensis, Festuca ovina, Dactylis glomerate und Cynodon dactylon im Gewächshaus angesät und nach Bedarf bewässert. Die aufgelaufenen Gräser werden wöchentlich bis auf 4 cm Höhe zurückgeschnitten und ca. 50 Tage nach der Aussaat und einen Tag nach dem letzten Schnitt mit der wässrigen Spritzbrühe eines Wirkstoffes der Formel I besprüht. Die Wirkstoffmenge beträgt umgerechnet bis zu 100 g Aktivsubstanz pro Hektar. 21 Tage nach Applikation wird das Wachstum der Gräser beurteilt.

Die Verbindungen der Formel I gemäss Tabelle 1 bis 8 bewirken eine Reduzierung des Neuzuwachses von um 10-30 % im Vergleich zur unbehandelten Kontrolle.

**Patentansprüche**

1. N-(2,2-Dioxo-1,2-benzoxathiin-8-ylsulfonyl)-N'-pyrimidinyl-, N'-triazolyl- oder N'-triazinyl-harnstoffe der Formel I

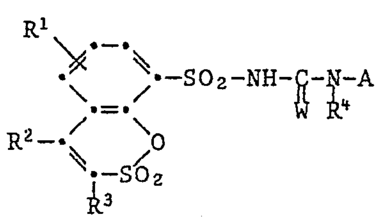

(I)

worin

$R^1$ Wasserstoff, Halogen, Nitro, $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkoxycarbonyl, $C_1$-$C_4$-Alkythio, $C_1$-$C_4$-Alkylsulfinyl, $C_1$-$C_4$-Alkylsulfonyl oder $C_2$-$C_5$-Alkoxyalkoxy,

$R^2$ und $R^3$ unabhängig voneinander Wasserstoff oder $C_1$-$C_4$-Alkyl,

$R^4$ Wasserstoff, Methyl oder Aethyl,

W Sauerstoff oder Schwefel, und

A einen Rest

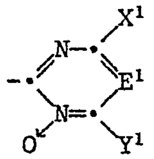

A1

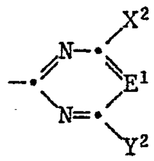

A2

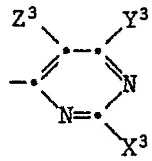

A3

,

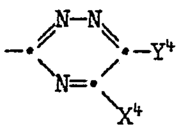

A4

,

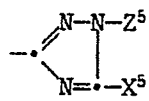

A5

,

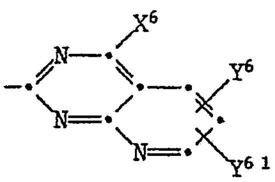

A6

,

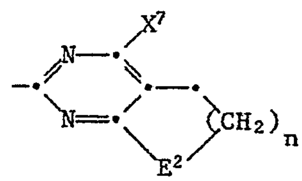

A7

oder

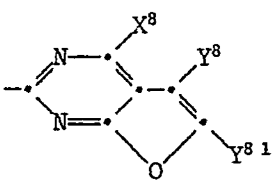

A8

bedeuten, wobei
$X^1$, $X^2$, $X^3$, $X^4$, $X^5$, $X^6$, $X^7$, $X^8$ und $Y^1$ unabhängig voneinander für Halogen, $C_1$-$C_4$-Halogenalkoxy, $C_1$-$C_4$-Halogenalkyl, $C_1$-$C_4$-Alkoxy, $X_1$-$C_4$-Alkyl, Dimethylamino, Methylamino, Aethylamino, Amino oder $C_2$-$C_4$-Alkoxyalkyl,
$E^1$ für Stickstoff oder die Methinbrücke,
$E^2$ für Sauerstoff oder die Methylenbrücke,
$Y^2$ für Cyclopropyl, Dimethoxymethyl, Diäthoxymethyl

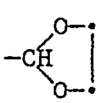

oder

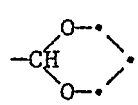

,

$Y^3$ für Methyl, Methoxy, Aethyl, Aethoxy, $C_1$-$C_2$-Halogenalkyl oder $C_1$-$C_2$-Halogenalkoxy, Dimethylamino, Methylamino,
$Y^4$ für Wasserstoff, Methoxy, Aethoxy, Halogen oder $C_1$-$C_4$-Alkyl,
$Y^6$ und $Y^{61}$ unabhängig voneinander für Wasserstoff, $C_1$-$C_4$-Alkyl, Halogen, Methoxy oder Methylthio,
$Y^8$ und $Y^{81}$ unabhängig voneinander für Wasserstoff oder Methyl,
n für eins oder zwei,
$Z^3$ für Wasserstoff, Methyl, Aethyl, Methoxy, Aethoxy, Methoxycarbonyl, Aethoxycarbonyl, Halogen, Cyan, Nitro, Methylthio, Methylsulfinyl oder Methylsulfonyl,
$Z^3$ und $Y^3$ zusammen für eine $C_2$-$C_4$-Alkylenbrücke oder eine durch Sauerstoff unterbrochene $C_2$-$C_4$-Alkylenbrücke, und
$Z^5$ für Methyl oder Aethyl stehen,
sowie den Salzen dieser Verbindungen; mit der Massgabe, dass $R^3$ $C_1$-$C_4$-Alkyl bedeutet wenn A für einen der Reste A2 oder A5 steht.

2. Verbindungen gemäss Anspruch 1, dadurch gekennzeichnet, dass $R^1$ und $R^2$ Wasserstoff und $R^3$

Methyl bedeuten.

3. Verbindungen gemäss Anspruch 1 oder 2, dadurch gekennzeichnet, das W Sauerstoff bedeutet.

4. Verbindungen gemäss Anspruch 1, dadurch gekennzeichnet,

a) dass W für Sauerstoff, $R^4$ für Wasserstoff und A für die Gruppe A1 stehen, wobei $X^1$ und $Y^1$ unabhängig voneinander je $C_1$-$C_4$-Alkyl oder $C_1$-$C_4$-Alkoxy bedeutet oder

b) dass W für Sauerstoff, $R^4$ für Wasserstoff und A für die Gruppe A2 stehen, wobei $Y^2$ Cyclopropyl bedeutet oder

c) dass W für Sauerstoff, $R^4$ für Wasserstoff und A für die Gruppe A3 stehen, wobei $Z^3$ Halogen, Methyl oder Aethyl bedeutet oder

d) dass W für Sauerstoff, $R^4$ für Wasserstoff und A für die Gruppe A3 stehen, wobei $Z^3$ Halogen und $Y^3$ $C_1$-$C_2$-Alkoxy oder $C_1$-$C_2$-Alkyl bedeuten oder

e) dass W für Sauerstoff, $R^4$ für Wasserstoff und A für die Gruppe A5 stehen, wobei $X^5$ $C_1$-$C_4$-Alkoxy und $Z^5$ Methyl oder Aethyl bedeuten oder

f) dass W für Sauerstoff, $R^4$ für Wasserstoff und A für die Gruppe A4 stehen, wobei $X^4$ $C_1$-$C_4$-Alkyl oder $C_1$-$C_4$-Alkoxy bedeutet.

5. N-(3-Methyl-2,2-dioxo-1,2-benzoxathiin-8-ylsulfonyl)-N'-(2,6-Dimethoxy-5-chlor-pyrimidin-4-yl)-harnstoff
oder
N-(3-Methyl-2,2-dioxo-1,2-benzoxathiin-8-ylsulfonyl)-N'-(2-methoxy-5-brom-6-methyl-pyrimidin-4-yl)-harnstoff gemäss Anspruch 1.

6. Verfahren zur Herstellung der Verbindungen der Formel I nach Anspruch 1, dadurch gekennzeichnet, dass man

a) ein Sulfonamid der Formel II

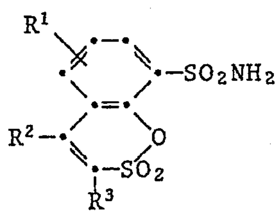

(II),

worin $R^1$, $R^2$ und $R^3$ die unter Formel I gegebene Bedeutung haben, in Gegenwart einer Base mit einem Carbamat der Formel III

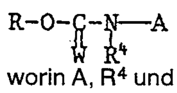

(III),

worin A, $R^4$ und W die unter Formel I gegebene Bedeutung haben und R für Phenyl, $C_1$-$C_4$-Alkyl oder substituiertes Phenyl steht, umsetzt und gegebenenfalls in ein Salz überführt oder

b) ein Sulfonylcarbamat der Formel IV

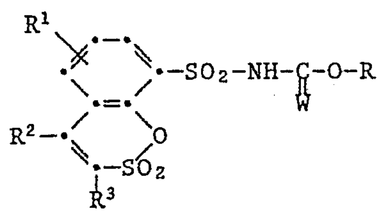

(IV),

worin $R^1$, $R^2$, $R^3$ und W die unter Formel I gegebene Bedeutung haben und R für Phenyl, $C_1$-$C_4$-Alkyl oder substituiertes Phenyl steht, mit einem Amin der Formel V

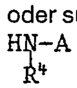

(V),

worin A und $R^4$ die unter Formel I gegebene Bedeutung haben, umsetzt und gegebenenfalls in ein Salz überführt oder

c) ein Sulfonylisocyanat der Formel VI

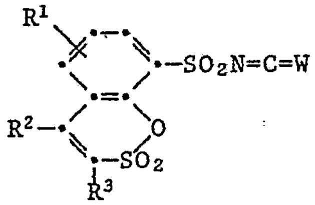

(VI),

worin $R^1$, $R^2$, $R^3$ und W die unter Formel I gegebene Bedeutung haben, mit einem Amin der Formel V

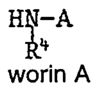

(V),

worin A und $R^4$ die unter Formel I gegebene Bedeutung haben, umsetzt und gegebenenfalls in ein Salz überführt.

7. Ein herbizides und den Pflanzenwuchs hemmendes Mittel, dadurch gekennzeichnet, dass es neben Träger- und/oder anderen Zuschlagstoffen als Wirkstoff mindestens einen substituierten Sulfonylharnstoff der Formel I, gemäss einem der Ansprüche 1 bis 5, enthält.

8. Verfahren zur Bekämpfung unerwünschten Pflanzenwachstums, dadurch gekennzeichnet, dass man einen Wirkstoff der Formel I, gemäss einem der Ansprüche 1 bis 5, oder ein diesen Wirkstoff enthaltendes Mittel gemäss Anspruch 7 in einer wirksamen Menge auf die Pflanzen oder deren Lebensraum appliziert.

9. Verfahren zur Hemmung des Pflanzenwachstums, dadurch gekennzeichnet, dass man einen Wirkstoff der Formel I, gemäss einem der Ansprüche 1 bis 5, oder ein diesen Wirkstoff enthaltendes Mittel gemäss Anspruch 7 in einer wirksamen Menge auf die Pflanzen oder deren Lebensraum appliziert.

10. Verfahren zur Beeinflussung des Pflanzenwachstums zum Zweck der Ertragssteigerung, dadurch gekennzeichnet, dass man einen Wirkstoff der Formel I, gemäss einem der Ansprüche 1 bis 5, oder ein diesen Wirkstoff enthaltendes Mittel gemäss Anspruch 7 in einer wirksamen Menge auf die Pflanzen oder deren Lebensraum appliziert.

11. Vermehrungsgut, welches mit einer herbizid oder pflanzenwuchsregulatorisch wirksamen Menge einer Verbindung der Formel I gemäss einem der Ansprüche 1 bis 5 behandelt worden ist.

Patentansprüche für den folgenden Vertragsstaat: ES

1. Verfahren zur Herstellung von Verbindungen der Formel I

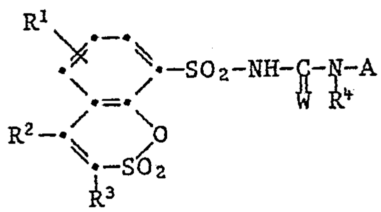

(I)

worin

$R^1$ Wasserstoff, Halogen, Nitro, $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkoxycarbonyl, $C_1$-$C_4$-Alkythio, $C_1$-$C_4$-Alkylsulfinyl, $C_1$-$C_4$-Alkylsulfonyl oder $C_2$-$C_5$-Alkoxyalkoxy,
$R^2$ und $R^3$ unabhängig voneinander Wasserstoff oder $C_1$-$C_4$-Alkyl,
$R^4$ Wasserstoff, Methyl oder Aethyl,
W Sauerstoff oder Schwefel, und
A einen Rest

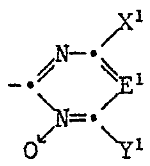

A1

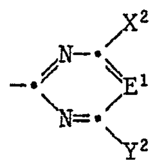

A2

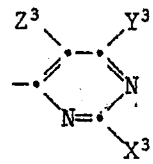

A3 ,

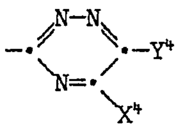

A4 ,

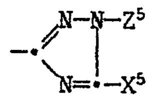

A5 ,

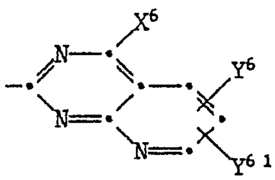

A6 ,

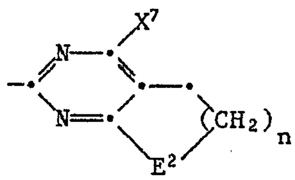

A7 oder

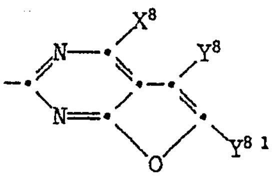

A8

bedeuten, wobei
$X^1$, $X^2$, $X^3$, $X^4$, $X^5$, $X^6$, $X^7$, $X^8$ und $Y^1$ unabhängig voneinander für Halogen, $C_1$-$C_4$-Halogenalkoxy, $C_1$-$C_4$-Halogenalkyl, $C_1$-$C_4$-Alkoxy, $C_1$-$C_4$-Alkyl, Dimethylamino, Methylamino, Aethylamino, Amino oder $C_2C_4$-Alkoxyalkyl,
$E^1$ für Stickstoff oder die Methinbrücke,
$E^2$ für Sauerstoff oder die Methylenbrücke,
$Y^2$ für Cyclopropyl, Dimethoxymethyl, Diäthoxymethyl

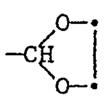 oder 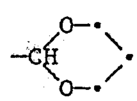 ,

$Y^3$ für Methyl, Methoxy, Aethyl, Aethoxy, $C_1$-$C_2$-Halogenalkyl oder $C_1$-$C_2$-Halogenalkoxy, Dimethylamino, Methylamino,
$Y^4$ für Wasserstoff, Methoxy, Aethoxy, Halogen oder $C_1$-$C_4$-Alkyl,
$Y^6$ und $Y^{61}$ unabhängig voneinander für Wasserstoff, $C_1$-$C_4$-Alkyl, Halogen, Methoxy oder Methylthio,
$Y^8$ und $Y^{81}$ unabhängig voneinander für Wasserstoff oder Methyl,
n für eins oder zwei,
$Z^3$ für Wasserstoff, Methyl, Aethyl, Methoxy, Aethoxy, Methoxycarbonyl, Aethoxycarbonyl, Halogen, Cyan, Nitro, Methylthio, Methylsulfinyl oder Methylsulfonyl,
$Z^3$ und $Y^3$ zusammen für eine $C_2$-$C_4$-Alkylenbrücke oder eine durch Sauerstoff unterbrochene $C_2$-$C_4$-Alkylenbrücke, und
$Z^5$ für Methyl oder Aethyl stehen,
sowie den Salzen dieser Verbindungen; mit der Massgabe, dass $R^3$ $C_1$-$C_4$-Alkyl bedeutet wenn A für einen der Reste A2 oder A5 steht, dadurch gekennzeichnet, dass man

a) ein Sulfonamid der Formel II

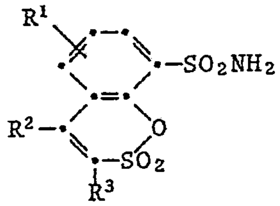

(II),

worin $R^1$, $R^2$ und $R^3$ die unter Formel I gegebene Bedeutung haben, in Gegenwart einer Base mit einem Carbamat der Formel III

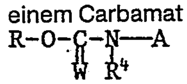

(III),

worin A, $R^4$ und W die unter Formel I gegebene Bedeutung haben und R für Phenyl, $C_1$-$C_4$-Alkyl oder substituiertes Phenyl steht, umsetzt und gegebenenfalls in ein Salz überführt oder

b) ein Sulfonylcarbamat der Formel IV

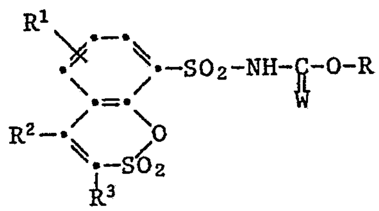

(IV),

worin $R^1$, $R^2$, $R^3$ und W die unter Formel I gegebene Bedeutung haben und R für Phenyl, $C_1$-$C_4$-Alkyl oder substituiertes Phenyl steht, mit einem Amin der Formel V

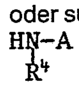

(V),

worin A und $R^4$ die unter Formel I gegebene Bedeutung haben, umsetzt und gegebenenfalls in ein Salz überführt oder

c) ein Sulfonylisocyanat der Formel VI

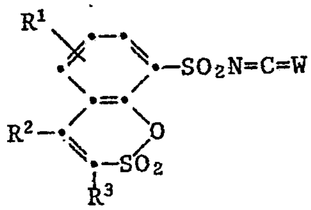

(VI),

worin $R^1$, $R^2$, $R^3$ und W die unter Formel I gegebene Bedeutung haben, mit einem Amin der Formel V

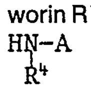

(V),

worin A und $R^4$ die unter Formel I gegebene Bedeutung haben, umsetzt und gegebenenfalls in ein Salz überführt.

2. Verfahren gemäss Anspruch 1 zur Herstellung von Verbindungen der Formel I, worin $R^1$ und $R^2$ Wasserstoff und $R^3$ Methyl bedeuten.

3. Verfahren gemäss Anspruch 1 oder 2 zur Herstellung von Verbindungen der Formel I, worin W Sauerstoff bedeutet.

4. Verfahren gemäss Anspruch 1 zur Herstellung von Verbindungen der Formel I, worin

a) W für Sauerstoff, $R^4$ für Wasserstoff und A für die Gruppe A1 stehen, wobei $X^1$ und $Y^1$ unabhängig voneinander je $C_1$-$C_4$-Alkyl oder $C_1$-$C_4$-Alkoxy bedeutet oder

b) W für Sauerstoff, $R^4$ für Wasserstoff und A für die Gruppe A2 stehen, wobei $Y^2$ Cyclopropyl bedeutet oder

c) W für Sauerstoff, $R^4$ für Wasserstoff und A für die Gruppe A3 stehen, wobei $Z^3$ Halogen, Methyl oder Aethyl bedeutet oder

d) W für Sauerstoff, $R^4$ für Wasserstoff und A für die Gruppe A3 stehen, wobei $Z^3$ Halogen und $Y^3$ $C_1$-$C_2$-Alkoxy oder $C_1$-$C_2$-Alkyl bedeuten oder

e) W für Sauerstoff, $R^4$ für Wasserstoff und A für die Gruppe A5 stehen, wobei $X^5$ $C_1$-$C_4$-Alkoxy und $Z^5$ Methyl oder Aethyl bedeuten oder

f) W für Sauerstoff, $R^4$ für Wasserstoff und A für die Gruppe A4 stehen, wobei $X^4$ $X_1$-$C_4$-Alkyl oder $C_1$-$C_4$-Alkoxy bedeutet.

5. Verfahren gemäss Anspruch 1 zur Herstellung von N-(3-Methyl-2,2-dioxo-1,2-benzoxathiin-8-ylsulfonyl)-N'-(2,6-Dimethoxy-5-chlor-pyrimidin-4-yl)-harnstoff
oder
N-(3-Methyl-2,2-dioxo-1,2-benzoxathiin-8-ylsulfonyl)-N'-(2-methoxy-5-brom-6-methyl-pyrimidin-4-yl)-harnstoff.

6. Verfahren zur Bekämpfung unerwünschten Pflanzenwachstums, dadurch gekennzeichnet, dass man einen Wirkstoff der Formel I

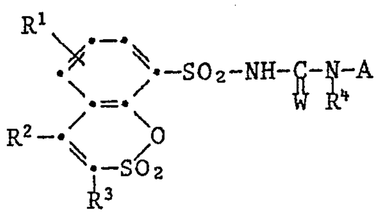

(I)

worin
$R^1$ Wasserstoff, Halogen, Nitro, $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkoxycarbonyl, $C_1$-$C_4$-Alkythio, $C_1$-$C_4$-Alkylsulfinyl, $C_1$-$C_4$-Alkylsulfonyl oder $C_2$-$C_5$-Alkoxyalkoxy,
$R^2$ und $R^3$ unabhängig voneinander Wasserstoff oder $C_1$-$C_4$-Alkyl,
$R^4$ Wasserstoff, Methyl oder Aethyl,
W Sauerstoff oder Schwefel, und
A einen Rest

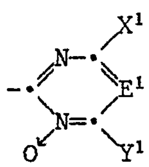

A1

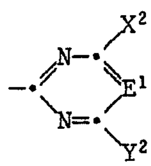

A2

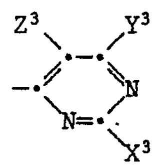

A3

,

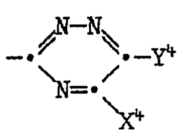

A4

,

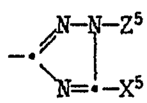

A5

,

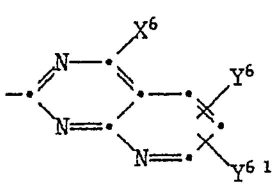

A6

,

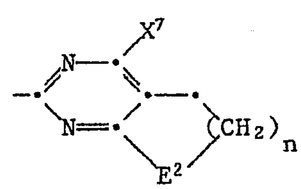

A7

oder

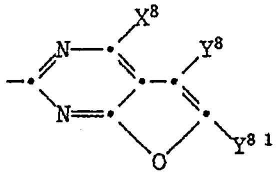

A8

bedeuten, wobei
$X^1$, $X^2$, $X^3$, $X^4$, $X^5$, $X^6$, $X^7$, $X^8$ und $Y^1$ unabhängig voneinander für Halogen, $C_1$-$C_4$-Halogenalkoxy, $C_1$-$C_4$-Halogenalkyl, $C_1$-$C_4$-Alkoxy, $C_1$-$C_4$-Alkyl, Diemthylamino, Methylamino, Aethylamino, Amino oder $C_2$-$C_4$-Alkoxyalkyl,
$E^1$ für Stickstoff oder die Methinbrücke,

$E^2$ für Sauerstoff oder die Methylenbrücke,
$Y^2$ für Cyclopropyl, Dimethoxymethyl, Diäthoxymethyl

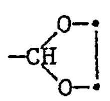 oder 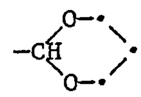 ,

$Y^3$ für Methyl, Methoxy, Aethyl, Aethoxy, $C_1$-$C_2$-Halogenalkyl oder $C_1$-$C_2$-Halogenalkoxy, Dimethylamino, Methylamino,
$Y^4$ für Wasserstoff, Methoxy, Aethoxy, Halogen oder $C_1$-$C_4$ Alkyl,
$Y^6$ und $Y^{61}$ unabhängig voneinander für Wasserstoff, $C_1$-$C_4$-Alkyl, Halogen, Methoxy oder Methylthio,
$Y^8$ und $Y^{81}$ unabhängig voneinander für Wasserstoff oder Methyl,
n für eins oder zwei,
$Z^3$ für Wasserstoff, Methyl, Aethyl, Methoxy, Aethoxy, Methoxycarbonyl, Aethoxycarbonyl, Halogen, Cyan, Nitro, Methylthio, Methylsulfinyl oder Methylsulfonyl,
$Z^3$ und $Y^3$ zusammen für eine $C_2$-$C_4$-Alkylenbrücke oder eine durch Sauerstoff unterbrochene $C_2$-$C_4$-Alkylenbrücke, und
$Z^5$ für Methyl oder Aethyl stehen,
sowie den Salzen dieser Verbindungen; mit der Massgabe, dass $R^3$ $C_1$-$C_4$-Alkyl bedeutet wenn A für einen der Reste A2 oder A5 steht in einer wirksamen Menge auf die Pflanzen oder deren Lebensraum appliziert.

7. Verfahren zur Hemmung des Pflanzenwachstums, dadurch gekennzeichnet, dass man einen Wirkstoff der Formel I,

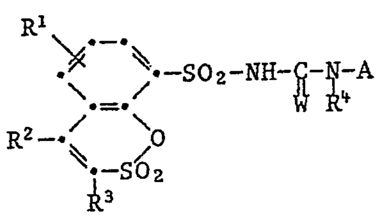 (I)

worin
$R^1$ Wasserstoff, Halogen, Nitro, $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkoxycarbonyl, $C_1$-$C_4$-Alkythio, $C_1$-$C_4$-Alkylsulfinyl, $C_1$-$C_4$-Alkylsulfonyl oder $C_2$-$C_5$-Alkoxyalkoxy,
$R^2$ und $R^3$ unabhängig voneinander Wasserstoff oder $C_1$-$C_4$-Alkyl,
$R^4$ Wasserstoff, Methyl oder Aethyl,
W Sauerstoff oder Schwefel, und
A einen Rest

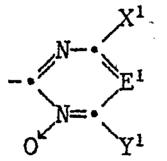

A1

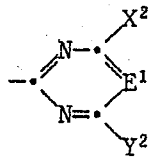

A2

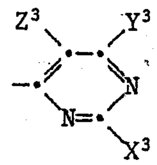

A3

,

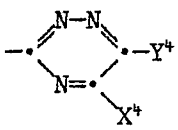

A4

,

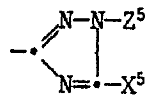

A5

,

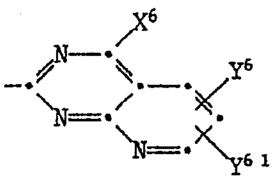

A6

,

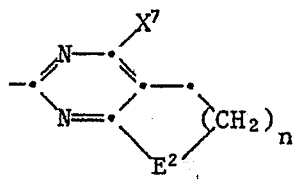

A7

oder

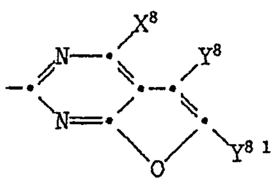

A8

bedeuten, wobei
$X^1$, $X^2$, $X^3$, $X^4$, $X^5$, $X^6$, $X^7$, $X^8$ und $Y^1$ unabhängig voneinander für Halogen, $C_1$-$C_4$-Halogenalkoxy, $C_1$-$C_4$-Halogenalkyl, $C_1$-$C_4$-Alkoxy, $C_1$-$C_4$-Alkyl, Dimethylamino, Methylamino, Aethylamino, Amino oder $C_2$-$C_4$-Alkoxyalkyl,
$E^1$ für Stickstoff oder die Methinbrücke,
$E^2$ für Sauerstoff oder die Methylenbrücke,
$Y^2$ für Cyclopropyl, Dimethoxymethyl, Diäthoxymethyl

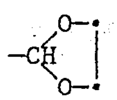 oder 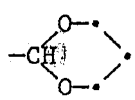 ,

$Y^3$ für Methyl, Methoxy, Aethyl, Aethoxy, $C_1$-$C_2$-Halogenalkyl oder $C_1$-$C_2$-Halogenalkoxy, Dimethylamino, Methylamino,
$Y^4$ für Wasserstoff, Methoxy, Aethoxy, Halogen oder $C_1$-$C_4$-Alkyl,
$Y^6$ und $Y^{61}$ unabhängig voneinander für Wasserstoff, $C_1$-$C_4$-Alkyl, Halogen, Methoxy oder Methylthio,
$Y^8$ und $Y^{81}$ unabhängig voneinander für Wasserstoff oder Methyl,
n für eins oder zwei,
$Z^3$ für Wasserstoff, Methyl, Aethyl, Methoxy, Aethoxy, Methoxycarbonyl, Aethoxycarbonyl, Halogen, Cyan, Nitro, Methylthio, Methylsulfinyl oder Methylsulfonyl,
$Z^3$ und $Y^3$ zusammen für eine $C_2$-$C_4$-Alkylbrücke oder eine durch Sauerstoff unterbrochene $C_2$-$C_4$-Alkylenbrücke, und
$Z^5$ für Methyl oder Aethyl stehen,
sowie den Salzen dieser Verbindungen; mit der Massgabe, dass $R^3$ $C_1$-$C_4$-Alkyl bedeutet wenn A für einen der Reste A2 oder A5 steht in einer wirksamen Menge auf die Pflanzen oder deren Lebensraum appliziert.

8. Verfahren zur Beeinflussung des Pflanzenwachstums zum Zweck der Ertragssteigerung, dadurch gekennzeichnet, dass man einen Wirkstoff der Formel I,

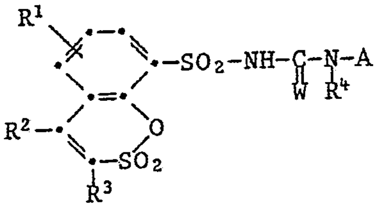

(I)

worin

$R^1$ Wasserstoff, Halogen, Nitro, $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkoxycarbonyl, $C_1$-$C_4$-Alkythio, $C_1$-$C_4$-Alkylsulfinyl, $C_1$-$C_4$-Alkylsulfonyl oder $C_2$-$C_5$-Alkoxyalkoxy,

$R^2$ und $R^3$ unabhängig voneinander Wasserstoff oder $C_1$-$C_4$-Alkyl,

$R^4$ Wasserstoff, Methyl oder Aethyl,

W Sauerstoff oder Schwefel, und

A einen Rest

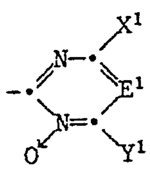

A1

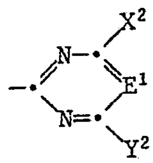

A2

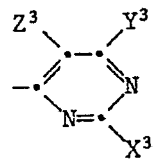

A3

,

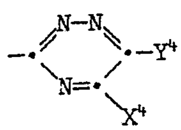

A4

,

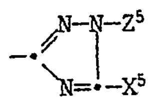

A5

,

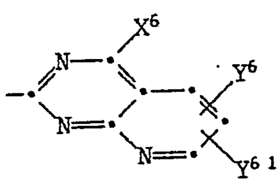

A6

,

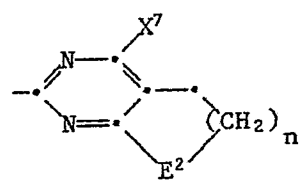

A7

oder

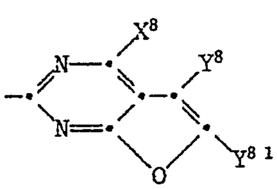

A8

bedeuten, wobei

$X^1$, $X^2$, $X^3$, $X^4$, $X^5$, $X^6$, $X^7$, $X^8$ und $Y^1$ unabhängig voneinander für Halogen, $C_1$-$C_4$-Halogenalkoxy, $C_1$-$C_4$-Halogenalkyl, $C_1$-$C_4$-Alkoxy, $C_1$-$C_4$-Alkyl, Diemthylamino, Methylamino, Aethylamino, Amino oder $C_2$-$C_4$-Alkoxyalkyl,

$E^1$ für Stickstoff oder die Methinbrücke,

$E^2$ für Sauerstoff oder die Methylenbrücke,

$Y^2$ für Cyclopropyl, Dimethoxymethyl, Diäthoxymethyl

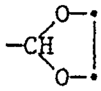 oder 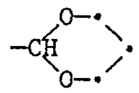 ,

$Y^3$ für Methyl, Methoxy, Aethyl, Aethoxy, $C_1$-$C_2$-Halogenalkyl oder $C_1$-$C_2$-Halogenalkoxy, Dimethylamino, Methylamino
$Y^4$ für Wasserstoff, Methoxy, Aethoxy, Halogen oder $C_1$-$C_4$-Alkyl,
$Y^6$ und $Y^{61}$ unabhängig voneinander für Wasserstoff, $C_1$-$C_4$-Alkyl, Halogen, Methoxy oder Methylthio,
$Y^8$ und $Y^{81}$ unabhängig voneinander für Wasserstoff oder Methyl,
n für eins oder zwei,
$Z^3$ für Wasserstoff, Methyl, Aethyl, Methoxy, Aethoxy, Methoxycarbonyl, Aethoxycarbonyl, Halogen, Cyan, Nitro, Methylthio, Methylsulfinyl oder Methylsulfonyl,
$Z^3$ und $Y^3$ zusammen für eine $C_2$-$C_4$-Alkylenbrücke oder eine durch Sauerstoff unterbrochene $C_2$-$C_4$-Alkylenbrücke, und
$Z^5$ für Methyl oder Aethyl stehen,
sowie den Salzen dieser Verbindungen; mit der Massgabe, dass $R^3$ $C_1$-$C_4$-Alkyl bedeutet wenn A für einen der Reste A2 oder A5 steht in einer wirksamen Menge auf die Pflanzen oder deren Lebensraum appliziert.

Europäisches Patentamt

# EUROPÄISCHER RECHERCHENBERICHT

Nummer der Anmeldung

EP 87 81 0689

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.3) |
|---|---|---|---|
| X,D | EP-A-0 128 116 (CIBA-GEIGY AG)<br>* Seite 28, Tabelle 5, Verbindungen Nr. 5.48-5.51; Ansprüche 1-6, 17-26 *<br>--- | 1-4,6-10 | C 07 D 411/12<br>C 07 D 411/14<br>C 07 D 471/04<br>C 07 D 491/04<br>A 01 N 47/36 //<br>(C 07 D 471/04<br>C 07 D 239:00<br>C 07 D 221:00 )<br>(C 07 D 491/04<br>C 07 D 307:00<br>C 07 D 239:00 ) |
| X,D | EP-A-0 107 979 (E.I. DU PONT DE NEMOURS AND CO.)<br>* Seite 164, Tabelle 27b, Zeilen 10-13; Seite 166, Tabelle 28b, Zeilen 24, 25; Seite 168, Tabelle 29b, Zeilen 26, 27; Ansprüche 1-4, 28-30, 32, 33 * | 1,3,6-10 | |
| A | * Seite 129, Tabelle 19a, Zeilen 25-35; Seite 172, Tabelle 30b, Zeilen 10-12 *<br>--- | 1 | |
| A | EP-A-0 168 135 (E.I. DU PONT DE NEMOURS AND CO.)<br>* Seite 22, Tabelle II, Zeile 22; Ansprüche 1-4, 7-11 *<br>----- | 1,6-10 | |

RECHERCHIERTE SACHGEBIETE (Int. Cl.3)

C 07 D 411/00
C 07 D 471/00
C 07 D 491/00
A 01 N 47/00

Der vorliegende Recherchenbericht wurde für alle Patentansprüche erstellt

| Recherchenort | Abschlußdatum der Recherche | Prüfer |
|---|---|---|
| BERLIN | 29-02-1988 | VAN AMSTERDAM L.J.P. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)